# EUROPEAN PATENT SPECIFICATION

(11) **EP 3 003 050 B1**
(45) Date of publication and mention of the grant of the patent: **26.06.2019**
(21) Application number: 14736972.2
(22) Date of filing: 27.05.2014
(51) Int. Cl.: A01N 63/04, C12N 1/14, A01P 3/00

(54) **NON-TOXIGENIC STRAIN OF ASPERGILLUS FLAVUS**
NICHT-TOXIGENER STAMM VON ASPERGILLUS FLAVUS
SOUCHE NON TOXINOGÈNE D'ASPERGILLUS FLAVUS

(30) Priority: 27.05.2013 IT RM20130305
(43) Date of publication of application: 13.04.2016
(73) Proprietor: Universita' Cattolica Del Sacro Cuore, 20123 Milano Mi (IT)
(72) Inventor: BATTILANI, Paola, I-29122 Parma PR (IT)
(74) Representative: Predazzi, Valentina
(86) International application number: PCT/IB2014/061751
(87) International publication number: WO 2014/191917

(56) References cited:
- US-B1- 6 306 386
- US-B1- 8 173 179
- DATABASE WPI Week 200931 Thomson Scientific, London, GB; AN 2009-F45188 XP002712518, & CN 101 363 006 A (UNIV ZHEJIANG) 11 February 2009 (2009-02-11)
- ANTONIO MAURO ET AL: "Structure of an Aspergillus flavus population from maize kernels in northern Italy", INTERNATIONAL JOURNAL OF FOOD MICROBIOLOGY, vol. 162, no. 1, 1 March 2013 (2013-03-01) , pages 1-7, XP055078412, ISSN: 0168-1605, DOI: 10.1016/j.ijfoodmicro.2012.12.021

## Description

### TECHNICAL FIELD OF THE INVENTION

The present invention relates to a new non-toxigenic strain of *Aspergillus flavus* and also to compositions comprising this strain, and to the use thereof as a biocontrol agent against the contamination by aflatoxins produced by toxigenic strains of *Aspergillus flavus* and/or *Aspergillus parasiticus.* In addition, the present invention also provides a method for preventing and/or reducing and/or eliminating the contamination by aflatoxins produced by toxigenic strains of *Aspergillus flavus* and/or *Aspergillus parasiticus* in crops such as full field crops and/or in controlled environments, such as in tunnels or greenhouses and soilless crops.

### KNOWN PRIOR ART

*Aspergillus flavus* and *Aspergillus parasiticus* are fungi that produce mycotoxins, such as aflatoxins and cyclopiazonic acid. In particular, *Aspergillus flavus* and *Aspergillus parasiticus* can produce aflatoxins B₁ and B₂ and G₁ and G₂ in favourable environmental and nutritional conditions. Aflatoxin B₁ is the most toxic natural substance known and is classified by the International Agency for Research on Cancer (IARC, 2002. IARC Monographs on the Evaluation of the Carcinogenic Risks to Humans: Some Traditional Herbal Medicines, some Mycotoxins, Naphthalene and Styrene, vol. 82. IARC, Geneva, pp. 301-366.) as group 1, the class of highest risk, due to its demonstrated carcinogenic effect on humans and animals. Although aflatoxin B₂ is classified in group 1, there is limited evidence regarding its carcinogenic effect. The toxic action of aflatoxins manifests itself following ingestion of foods contaminated with these toxins with subsequent adverse effects, both acute and chronic.

*Aspergillus flavus* and *Aspergillus parasiticus* are able to infect various types of crops, such as maize crops, cotton crops and nuts, with subsequent contamination of the food products derived from such crops with aflatoxins. In addition, aflatoxins are also able to pass into animals fed with contaminated products. In particular, a transfer has been reported into milk obtained from cows and buffalo and into cheeses derived therefrom, in which a concentration of the toxins is observed depending both on the type of rennet and the aging period.

On the African continent, for example, the transfer of aflatoxins has contributed in recent years, specifically 2004 and 2005, to the deaths of more than 200 people by acute toxicity by aflatoxins ingested from highly contaminated maize (Probst et al., 2007. Outbreak of an acute aflatoxicosis in Kenya in 2004: identification of the causal agent. App. Environ. Microbiol. 73, 2762-2764).

Due to the high toxicity of aflatoxins, a regulation is currently in place in Europe (European Commission (EC), 2010. Commission Regulation No 165/2010 setting maximum levels for certain contaminants in foodstuffs as regards aflatoxins. Official Journal of the European Union L50, 8-12) that defines the maximum limits for the presence of aflatoxins in various products for human consumption. In particular, the specified regulation sets 2 values, one for aflatoxin B₁ (5.0 µg/kg) and one of the total sum of aflatoxins B₁, B₂, G₁ and G₂ (10 µg /kg), in maize intended for human use prior to purification and the maximum content of aflatoxin B₁ in milk (0.050 µg/kg). In addition EC Regulation 2003 (European Commission (EC), 2003. Commission Directive No 2003/100/EC on undesirable substances in animal feed. Official Journal of the European Union L285, 33-37) sets the maximum limits for the presence of aflatoxins in foodstuffs intended for animals (0.02 mg/kg) with certain restrictions for those based on milk (0.005 mg/kg).

Among the aflatoxins, B₁ is the most commonly present in contaminated foodstuffs. In particular in Italy, it has been observed that this aflatoxin represents 90% of the total aflatoxins identified in maize (Battilani P., Barbano C., Piva G. 2008. Aflatoxin B1 contamination in maize related to the aridity index in North Italy. World Mycotoxin J. 1, 449-456).

Currently, there are no direct means available in order to control the development of the fungus in the field. Guidelines have been drafted that define the best cultivation techniques for minimising the contaminations by aflatoxins and define criteria to follow in order to optimise the harvest, the drying and the storage of the grains so as to prevent the increase of the contaminations during these phases. However, in crops and geographical areas in which meteorological conditions favourable to aflatoxigenic fungi are observed, vigilant adherence to the guidelines does not guarantee production in accordance with legal requirements. In addition, the technical processes commonly applied do not make it possible to abate the contamination and there is a lack of effective detoxification systems authorised for application to the grain during the post-harvest period.

Among the proposed strategies for limiting the contamination by aflatoxins in crops intended for consumption, the most promising is based on the use of non-toxigenic strains of *Aspergillus flavus* or of strains of *Aspergillus flavus* that are unable to produce aflatoxins. The principle forming the basis of this strategy lies in the competitive exclusion between non-toxigenic strains of *Aspergillus flavus* and toxigenic strains (strains of *Aspergillus flavus* that produce aflatoxins), which in fact makes it possible in the field to limit the activity of the toxigenic strains and therefore the contamination by aflatoxins.

The use of non-toxigenic strains of *Aspergillus flavus* in the field as biocontrol agents that act by means of competitive exclusion was proposed in the USA, where there are currently 2 commercial strains in existence (*Aspergillus flavus* AF36 and AFLAGUARD®). In addition, a mixture of non-toxigenic strains of *A. flavus* is in the process of being registered in Africa (AFLASAFE™). Usually, the non-toxigenic strains to be used as bicontrol agents such as those specified above by way of example are selected from maize crops. The strains thus selected can then be used as bicontrol agents and also on various types of crops liable to infection by *Aspergillus flavus* and/or *Aspergillus parasiticus.*

However, the strains available in the USA or in Africa are not transferrable into Europe, including Italy. In fact, even irrespective of the regulations regarding the introduction of microorganisms originating from various countries, the adaptation to rather different agro-climatic environments, such as those in Africa and the USA, means that the available non-toxigenic strains are barely competitive towards the native strains. In fact, as observed in a work describing isolated strains in Italy (Giorni P., Magan N., Pietri A., Bertuzzi T., Battilani P. Studies on Aspergillus Section Flavi isolated in northern Italy from maize. International Journal of Food Microbiology, 2007, 113, 330-338), the Italian strains seem less thermophilic compared with those observed in other environments.

It should be added that, with use of such strains, it is not possible to rule out the risk that the toxigenicity of the strain used might be restored if the vegetative compatibility group (VGC) to which the strains belong are not checked beforehand, since *Aspergillus flavus* is able to exchange genetic material by fusion of hyphae with strains belonging to the same VCG. In other words, should toxigenic and anti-toxigenic strains belong to the same VCG and come into contact with one another, genetic material will be exchanged, with the non-toxigenic strain acquiring the ability to produce aflatoxins.

The object of the present invention is to overcome the disadvantages associated with the use of known non-toxigenic strains of *Aspergillus flavus* and to provide an alternative non-toxigenic strain of *Aspergillus flavus* to be used to prevent and/or eliminate partially or wholly the contamination by aflatoxins. This would appear to be all the more appropriate following the significant problems experienced in the agricultural crop of 2012, with a significant proportion of the maize produced in Italy, estimated at around 60%, being contaminated above the legal limits. The high contamination, attributable primarily to the meteorological conditions experienced, with high temperatures and a lack of rain during the summer period, is expected to be more likely to occur in the near future due to the current climate change, with subsequent problems for public health.

### SUMMARY OF THE INVENTION

The present invention relates to a new non-toxigenic strain of *Aspergillus flavus.* In particular, the inventor has found that such a strain can be used as a biocontrol agent against the contamination by aflatoxins, for example in field crops in Italy, surprisingly resulting in reductions of the contamination by *Aspergillus flavus* and/or by *Aspergillus parasiticus,* with advantages greater than those acknowledged for the known non-toxigenic strains of *Aspergillus flavus,* as will be demonstrated in the section below containing the examples.

The present invention is based on the observation that the currently commercially available strains, that is to say non-toxic strains of *Aspergillus flavus* native to the American territory (*Aspergillus flavus* AF36 and AFLAGUARD®) or mixtures of likewise non-toxic strains of *Aspergillus flavus* of African origin (AFLASAFE™), cannot be used in Italy for legislative reasons and also due to the presumed poor competitive ability, as indicated by Probst et al. (Probst, C., Bandyopadhyay, R., Price, L. E., Cotty, P. J. 2011. Identification of atoxigenic Aspergillus flavus isolates to reduce aflatoxin contamination of maize in Kenya. Plant Dis. 95, 212-218). In particular, the inventor of the present invention has selected a new non-toxigenic strain of *Aspergillus flavus,* native to the Italian territory, which has proven to be extremely effective as a bicontrol agent against the contamination by aflatoxins in Italy. Thus, an Italian non-toxigenic strain of *Aspergillus flavus* is described here for the first time, of which the use, in the Italian territory, makes it possible to obtain crops and also post-harvest products in which the levels of aflatoxins are minimal. It follows that there is a high likelihood of obtaining crops and derived products having aflatoxin levels within the limits imposed by the European regulations on this topic (European Regulation 1881/2006, updated by European Regulation 165/2010 for products intended for human use and Directive 100/2003 for products intended for use in zoos), even in years in which the crops are at high risk.

In particular, the ability of the strain of *Aspergillus flavus* described here to prevent and/or eliminate wholly or in part the contamination by aflatoxins, such as aflatoxins B₁, B₂, G₁ and G₂, in various Italian agricultural crops is due to the combination of its genetic/biochemical characteristics, which include the following:
a) Italian origin, therefore in other words is a native Italian strain;
b) inability to produce aflatoxins and is therefore a non-toxigenic strain;
c) inability to produce cyclopiazonic acid (CPA);
d) vegetative compatibility group (VCG) IT6 or VCG, which has demonstrated the best diffusion over the Italian territory;
e) allele MAT1-1 at the locus for the gene mating type (MAT).

As demonstrated in the section below entitled "Examples", it has surprisingly been demonstrated that the strain of *Aspergillus flavus* described here is more effective than any other native Italian strain and as a bicontrol agent in agriculture. In fact, the non-toxigenic strain described here is able to reduce the aflatoxin content to an extent from 75% to 100% in tests on full field crops contaminated by *Aspergillus flavus* and/or *Aspergillus parasiticus.* In addition, with the method of pin bar inoculation of the ear, the strain of the present invention has proven to be able to reduce the content of aflatoxins in a percentage greater than 80% in ears contaminated by *Aspergillus flavus.* In particular, as demonstrated in the comparative test reported in Example 5, the results of which are summarised in Table 5, the method of pin bar inoculation of the ear with the strain of the invention demonstrated a tangible reduction of aflatoxin B₁ of approximately 93.2% in the field, whereas the non-toxigenic strain A2321 (seemingly effective in laboratory tests) was unable in the tests in the field to reduce the percentage of aflatoxin compared with the control.

In addition, the inventors of the present invention have also demonstrated (Example 6) how the strain described here has the ability to adapt better to different environmental and meteorological conditions, since it is able to compete and diffuse more effectively than the strain A2321. Advantageously, the strain described here can thus be used effectively also to reduce the contamination of aflatoxins in fields having rather diverse climatic/environmental characteristics.

The present description therefore relates to:
- a non-toxigenic strain of *Aspergillus flavus* having deposit number MUCL54911, filed on 13 May 2013 at *"The Belgian Co-ordinated Collections of Micro-organisms (BCCM*™*)* and/or derivatives thereof that maintain the characteristics of said strain disclosed in the present description;
- a composition comprising the above strain or derivatives thereof and one or more diluents and/or carriers:
- use of the strain MUCL54911 of or derivatives thereof or of the composition comprising same as a bicontrol agent against the contamination by aflatoxins produced by *Aspergillus flavus* and/or *Aspergillus parasiticus;*
- a method for preventing and/or reducing and/or eliminating the contamination by aflatoxins produced by toxigenic *Aspergillus flavus* and/or *Aspergillus parasiticus* in field crops comprising a step of distributing the strain MUCL54911 or derivatives thereof or the above composition to said crop;
- a matrix inoculated with the strain MUCL54911 according to claim 1 or derivatives or parts thereof as defined above.

### FIGURES

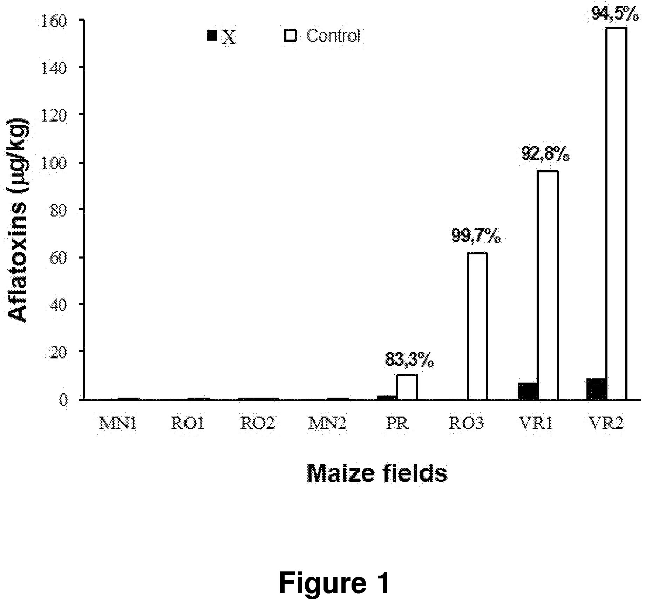

Figure 1 shows a graph illustrating the assessment of the full field efficacy of the non-toxigenic strain of *Aspergillus flavus* of the invention in reducing the content of aflatoxins. The numbers above the bars indicate the reduction in % obtained in the fields treated with the non-toxigenic strain of *Aspergillus flavus* having deposit number MUCL54911 compared with the untreated control.

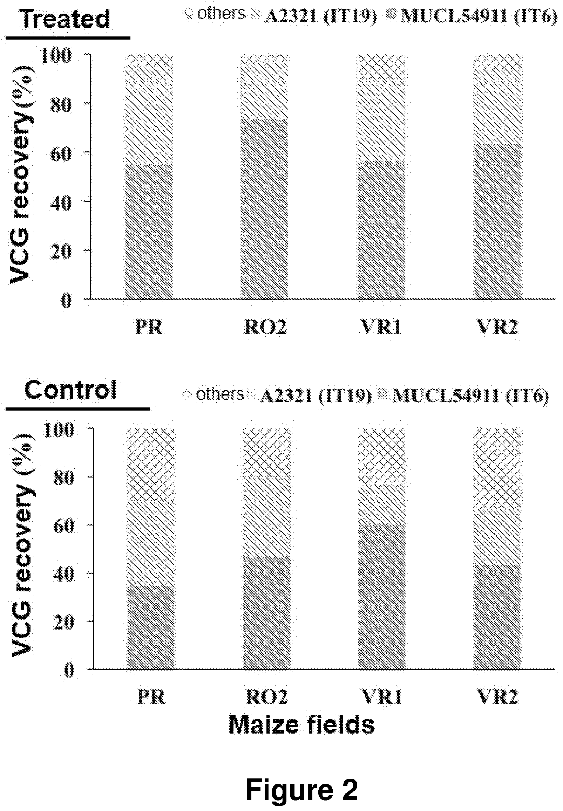

Figure 2 shows a graph illustrating the recovery in % of the strain having deposit number MUCL54911 (IT6), of strain A2321 (IT19), and of other vegetative compatibility groups in the treated and control sections of 4 fields where a reduction of the aflatoxin content was obtained.

### TABLES

Table 1 presents the data concerning the ability of non-toxigenic strains of *Aspergillus flavus,* compared with the strain of the invention (in bold), to reduce the production of aflatoxin B₁ in maize grain.

**Table 1**

| **Strain^{a}** | **VCG^{b}** | **mg/kg AFB1^{ce}** | | **Reduction (%)^{de}** | |
|---|---|---|---|---|---|
| A2321 | IT19 | 15 | H | 90 | A |
| A2103 | IT15 | 21 | GH | 85 | AB |
| A2090 | IT9 | 22 | FGH | 85 | ABC |
| A2066 | IT8 | 23 | EFGH | 85 | ABC |
| NRRL 21882 | n.a. | 24 | EFGH | 84 | ABC |
| **MUCL54911** | **IT6** | **25** | **EFGH** | **83** | **ABC** |
| A2088 | IT9 | 27 | EFG | 82 | ABC |
| A2105 | IT6 | 27 | DEFG | 81 | ABC |
| A2313 | IT23 | 28 | DEFG | 81 | ABCD |
| A2098 | IT22 | 29 | CDEFG | 80 | ABCD |
| A2096 | IT6 | 30 | CDEFG | 79 | BCD |
| A2102 | n.a. | 32 | BCDEFG | 78 | BCDE |
| A2319 | IT22 | 33 | BCDEFG | 78 | BCDE |
| A2049 | IT12 | 39 | BCDE | 73 | CDEF |
| A2320 | IT9 | 41 | BCDE | 72 | CDEF |
| A2087 | n.a. | 47 | BCD | 68 | DEF |
| A2322 | IT34 | 51 | BC | 65 | EF |
| A2323 | IT23 | 54 | BC | 62 | EF |
| A2100 | IT18 | 57 | B | 61 | F |
| AF13 | YV13 | 146 | A | 0 | G |

| | | | | | |
|---|---|---|---|---|---|
| ^{a} The non-toxigenic strains were inoculated with AF13 at a concentration of 1x10⁵ spores/ml. The strain NRRL21882 is the active component of the biopesticide AFLAGUARD®. The strain AF13 is a high producer of aflatoxins. ^{b} Vegetative Compatibility Group; n.a.= not available. ^{c} The concentration of aflatoxin B₁ was measured on the maize grain co-inoculated with the non-toxigenic and toxigenic strains after 7 days of incubation at 31°C. ^{d} Reduction of aflatoxin B₁ in % = [1- (aflatoxin B₁ in the co-inoculated grain/aflatoxin B₁ produced by AF13 alone)] x 100. ^{e} The letters indicate significant differences between the isolates (P<0.05). The combined data (P<0.05) of two replications of the experiment are shown in the table. | | | | | |

Table 2 presents the data concerning the ability of the strain of the invention (in bold), compared with another Italian non-toxigenic strain of *Aspergillus flavus* described previously in the literature, to reduce the concentration of aflatoxin B₁ produced by six Italian toxigenic strains of *Aspergillus flavus.*

**Table 2**

| **Strain^{a}** | **mg/kg AFB1^{bd}** | | **mg/kg AFB1^{bd}** | | | | **Reduction (%)^{cd}** | | | |
|---|---|---|---|---|---|---|---|---|---|---|
| | | | **MUCL54911** | | **A2321** | | **MUCL54911** | | **A2321** | |
| A2039 | 98 | A | **22** | **A** | 17 | A | **78** | **A** | 83 | A |
| A2062 | 35 | BC | **11** | **BC** | 8 | B | **68** | **B** | 78 | AB* |
| A2068 | 41 | B | **16** | **AB** | 16 | A | **60** | **B** | 61 | C |
| A2097 | 29 | C | **11** | **BC** | 8 | B | **61** | **B** | 71 | BC |
| A2295 | 23 | D | **8** | **CD** | 7 | BC | **65** | **B** | 71 | BC |
| A2300 | 17 | E | **7** | **D** | 5 | C | **60** | **B** | 72 | BC* |

| | | | | | | | | | | |
|---|---|---|---|---|---|---|---|---|---|---|
| ^{a} The non-toxigenic strains MUCL54911 and A2321 (described in the literature) were co-inoculated at the same time with toxigenic strains at a concentration of 1x10⁵ spores/ml. ^{b} The concentration (mg/kg) of aflatoxin B₁ (AFB1) of the maize grain was determined after 7 days of incubation at 31°C. ^{c} Reduction of aflatoxin B₁ in % = [1- (aflatoxin B₁ in the co-inoculated maize grain/aflatoxin B₁ produced by respective toxigenic strains)] x 100. ^{d} The letters indicate significant differences between the isolates (columns); * indicates differences between the non-toxigenic strains (rows). The combined data (P<0.05) from two independent replications of the experiment are shown. | | | | | | | | | | |

Table 3 presents the data concerning the influence of the non-toxigenic:toxigenic ratio between strains of *Aspergillus flavus* compared with the strain of the invention (in bold) in reducing the content of aflatoxin B₁ produced by a toxigenic strain.

**Table 3**

| **Strain^{a}** | **mg/kg AFB1^{cf}** | | | | **Reduction (%)^{df}** | | | | **Efficacy^{cf}** | | | |
|---|---|---|---|---|---|---|---|---|---|---|---|---|
| | **(1 : 4)^{b}** | | **(1 : 1)^{b}** | | **(1 : 4)^{b}** | | **(1 : 1)^{b}** | | **(1 : 4)^{b}** | | **(1 : 1)^{b}** | |
| A2066 | 86 | B | 44 | B* | 24 | D* | 61 | B | 1.20 | C | 1.21 | B |
| **MUCL54911** | **70** | **CD** | **31** | **B*** | **38** | **BC*** | **72** | **AB** | **1.90** | **B** | **1.45** | **AB** |
| A2090 | 76 | BCD | 37 | B* | 33 | BCD* | 67 | B | 1.66 | BC | 1.35 | AB* |
| A2103 | 68 | D | 34 | B* | 39 | AB* | 70 | AB | 1.97 | AB | 1.39 | AB* |
| A2321 | 56 | E | 20 | C* | 50 | A* | 83 | A | 2.52 | A | 1.65 | A* |
| NRRL21882 | 83 | BC | 40 | B* | 26 | CD* | 65 | B | 1.30 | C | 1.29 | B |
| Mean | 73 | | 34 | * | 35 | * | 70 | | 1.76 | | 1.39 | * |
| AF13 | 113 | A | 113 | A | ... | | ... | | ... | | ... | |

| | | | | | | | | | | | | |
|---|---|---|---|---|---|---|---|---|---|---|---|---|
| ^{a} The strain NRRL21882 is the active component of the biopesticide AFLAGUARD®. The strain AF13 is a high producer of aflatoxins and represents the positive control. The rest are Italian non-toxigenic strains. ^{b} The non-toxigenic (A) and toxigenic (T) strains were co-inoculated at the concentration of 1x10⁵ spores/ml in two ratios: (a) 1/5 non-toxigenic and 4/5 toxigenic (1:4) and (b) equal proportions (1:1). ^{c} The concentration (mg/kg) of aflatoxin B₁ (AFB1) of the maize grain was determined after 7 days of incubation at 31°C. ^{d} Reduction of aflatoxin B₁ in % (R) = [1- (aflatoxin B₁ in the co-inoculated maize grain/aflatoxin B₁ produced by the strain AF13 alone)] x 100. ^{e} The efficacy (E) was calculated with the following formula: E = R/(A/A+T); where R is the reduction in % of aflatoxin B₁ and the denominator represents the percentage of the non-toxigenic strain in each treatment. ^{f} The letters indicate significant differences between the isolates (columns); * indicates differences between the non-toxigenic strains (rows). The combined data (P<0.05) from two independent repetitions of the experiment are shown. | | | | | | | | | | | | |

Table 4 presents the data concerning the production of cyclopiazonic acid (CPA) on the maize grain and synthetic substrate (CZ) of strains of *Aspergillus flavus* compared with the strain of the invention shown in bold.

**Table 4**

| **Strain^{a}** | **mg/kg CPA^{b}** | |
|---|---|---|
| | **Maize^{c}** | **CZ^{e}** |
| **Control** | n.r. | n.r. |
| A2062 | 1108 | 9873 |
| A2066 | n.r. | n.r. |
| A2068 | 1165 | 7089 |
| **MUCL54911** | **n.r.** | **n.r.** |
| A2090 | n.r. | n.r. |
| A2092 | 718 | 7840 |
| A2103 | n.r. | n.r. |
| A2321 | n.r. | n.r. |

| | | |
|---|---|---|
| ^{a} Strains of *Aspergillus flavus.* Control: non-inoculated maize or CZ. ^{b} Concentration (mg/kg) of cyclopiazonic acid (CPA); n.r. = not revealed. ^{c} Maize = maize grain; CZ = synthetic substrate. | | |

**Table 5**

| **Year** | **Strain^{a}** | **Aflatoxin B₁ (µg/kg)** | **Reduction (%)^{b}** |
|---|---|---|---|
| 2012 | | | |
| | A2092 | 1415.4 | |
| | **MUCL54911** + A2092 | 96.2 | 93.2 |
| | A2321 + A2092 | 1381.9 | 2.4 |

| 2013 | | | |
|---|---|---|---|
| | A2092 | 132.9 | |
| | **MUCL54911** + A2092 | 2.3 | 98.3 |
| | A2321 + A2092 | 176.7 | n.o. |

| | | | |
|---|---|---|---|
| ^{a} Strains of *Aspergillus flavus.* ^{b} Reduction of aflatoxin B₁ in % = [1 - (aflatoxin B₁ in the co-inoculated maize grain/aflatoxin B₁ produced by the strain A2092 alone)] x 100; n.o. not obtained. | | | |

**Table 6 presents the haplotype of Italian non-toxigenic strains of Aspergillus flavus compared with the strain of the invention (in bold for 19 loci of microsatellites**

| | **Locus** | | | | | | | | | | | | | | | | | | |
|---|---|---|---|---|---|---|---|---|---|---|---|---|---|---|---|---|---|---|---|
| **Strain** | **AF8** | **AF11** | **AF13** | **AF14** | **AF17** | **AF18** | **AF22** | **AF28** | **AF31** | **AF33** | **AF34** | **AF42** | **AF43** | **AF53** | **AF54** | **AF55** | **AF63** | **AF64** | **AF66** |
| **MUCL 54911** | **166^{a}** | **135** | **141** | **169** | **367** | ****** | **144** | **119** | **312** | ****** | ****** | **150** | **399** | **131** | **161** | **181** | **127** | **161** | **271** |
| A2321 | 168 | 126 | 128 | 169 | 364 | ** | 144 | 119 | 312 | ** | ** | 143 | 399 | 131 | 161 | 172 | 127 | 161 | 269 |

| | | | | | | | | | | | | | | | | | | | |
|---|---|---|---|---|---|---|---|---|---|---|---|---|---|---|---|---|---|---|---|
| ^{a} Number of base pairs for each allele; ** allele not present. | | | | | | | | | | | | | | | | | | | |

### GLOSSARY

Strain of *Aspergillus flavus* MUCL54911. This term indicates the fungus having deposit number MUCL54911, filed on 13 May 2013 at *"The Belgian Co-ordinated Collections of Micro-organisms (BCCM*™*)"* at any moment of the life phase and also single cells thereof. The definition of *Aspergillus flavus* having deposit number MUCL54911 thus includes single fungus cells, the entire organism, and parts of the organism such as hyphae, spores, sclerotia and the like.
Strain inoculated on a matrix/support/substrate. The expression "strain inoculated on a matrix/support/substrate" in the present description means that parts of the fungus able to allow the growth thereof, for example the mycelium or spores of various type, are placed in contact with a matrix or substrate for the purpose of allowing the growth of the fungus on the matrix/substrate/support itself.
Vegetative compatibility (VC). The expression "Vegetative Compatibility" in the present invention, in accordance with that indicated in the scientific literature, means the ability of fungi, including *Aspergillus flavus,* to merge their own hyphae with those of other fungi belonging to the same species, thus forming stable heterokaryons (cells provided with a number of nuclei). The gene loci that regulate the vegetative compatibility are known as *vic* loci. Stable fusion between the hyphae is possible only between strains that have the same alleles in all the *vic* loci. Two strains that can merge their own hyphae are defined as strains belonging to the same Vegetative Compatibility Group (VCG) (Barros et al. 2006. Genetic diversity within Aspergillus flavus strains isolated from peanut-cropped soils in Argentina. Soil Biol.Biochem. 38:145-152).
Toxigenic. The term "toxigenic" in the present description means a strain of *Aspergillus flavus* or of *Aspergillus parasiticus* that produces aflatoxins and that is therefore a strain that forms toxic substances. Conversely, the term "non-toxigenic" refers always in the present description to a strain of *Aspergillus flavus* that is unable to produce aflatoxins inclusive of aflatoxin B₁ and that is thus a strain that is not toxigenic.
Aflatoxins. The term "aflatoxins" in the present description means secondary metabolites produced by various species of the fungus *Aspergillus,* including the species *Aspergillus flavus* and/or the species *Aspergillus parasiticus.* In particular, with reference to the aflatoxins produced by *Aspergillus flavus* and/or by *Aspergillus parasiticus,* these are indicated as aflatoxins B₁, B₂, G₁ and G₂. In the present description, unless indicated otherwise, the term "aflatoxin/s" refers indifferently to aflatoxin B₁, B₂, G₁, G₂ or mixtures thereof.
Derivatives. The term "derivatives" in the present description refers to strains of *Aspergillus flavus* derived, for example by mutation or transformation or crossbreeding, from the strain having deposit number MUCL54911, filed on 13 May 2013 at *"The Belgian Co-ordinated Collections of Micro-organisms (BCCM*™*)"* that maintain the characteristics claimed and described here of the strain of the description and that can therefore be used as the strain of the description.

The maintained characteristics include the following:
a) Italian origin;
b) inability to produce aflatoxins;
c) inability to produce cyclopiazonic acid (CPA);
d) vegetative compatibility group (VCG) IT6;
e) allele MAT1-1 at the locus for the gene mating type (MAT);
f) ability to reduce the aflatoxin content to an extent from 75% to 100% in tests on full field crops contaminated by *Aspergillus flavus* and/or *Aspergillus parasiticus;*
g) ability to reduce the content of aflatoxins in a percentage greater than 80% in cultures contaminated by *Aspergillus flavus* and/or *Aspergillus parasiticus* with the method of pin bar inoculation as described in the present application.

Native Italian. In the present description, the expression "native Italian" is to be understood, in accordance with that which is commonly known, as indicative of a strain of *Aspergillus flavus* originating from or that has been developed in the Italian territory in which it has been isolated.

### DETAILED DESCRIPTION OF THE INVENTION

The present invention relates to a new non-toxigenic strain of *Aspergillus flavus,* as filed on 13 May 2013 at *"The Belgian Co-ordinated Collections of Micro-organisms (BCCM*™*)"* in the name of the Università Cattolica del Sacro Cuore and assigned the deposit number MUCL54911 in accordance with the provisions of the Budapest Treaty of 28 April 1977, ratified by law n. 610 of 14 October 1985.

In particular, this strain has various characteristics including those specified below. It is a native Italian strain, that is to say a strain that, in so far as isolated from the harvests present in particular in Northern Italy, originates from or has been developed in the Italian geographical area.

The strain MUCL54911 is characterised in that it is a non-toxigenic strain or a strain of *Aspergillus flavus* devoid of the ability to produce aflatoxins. In particular, as reported in the prior art (Sweeney, M. J., Dobson, A. D. W. 1999. Molecular biology of mycotoxin biosynthesis. FEMS Microbiol. Lett. 175, 149-163), aflatoxins are synthesised in accordance with a synthetic pathway that involves at least 23 enzymatic conversions, which involve as many genes, in the strain MUCL54911 and is interrupted as soon as the entire cluster of genes involved in this synthesis has been deleted. Due to this genetic characteristic, the strain MUCL54911 is therefore a strain that produces neither aflatoxin B1 nor aflatoxin B2 and therefore is a non-toxigenic strain.

The strain MUCL54911 is also characterised in that it is devoid of the ability to produce cyclopiazonic acid (CPA) since a genetic deletion involves the genes involved in the production of CPA.

In addition, the strain MUCL54911 presents, as a genotype characteristic, the presence of the allele MAT1-1 at the locus for the gene mating type (MAT).

Lastly, the strain of the invention is also characterised in that it is able to reduce the content of aflatoxins, such as aflatoxin B₁, in a percentage from 75% to 100% in full field tests in crops contaminated by *Aspergillus flavus* and/or *Aspergillus parasiticus.* In particular, the content of aflatoxins in full field may be reduced by 75%, 76%, 77%, 78%, 80%, 81%, 82%, 83%, 84%, 85%, 86%, 87%, 88%, 89%, 90%, 91%, 92%, 93%, 94%, 95%, 96%, 97%, 98%, 99% or 100%.

In addition, the strain of the present invention has proven to be capable of reducing the content of aflatoxins in a percentage greater than 80%, 81%, 82%, 83%, 84%, 85%, 86% 87%, 88%, 89%, 90%, 91%, 92%, 93%, 94%, 95%, 96%, 97%, 98%, 99% and 100% in crops contaminated by *Aspergillus flavus* and/or *Aspergillus parasiticus* using the method of pin bar inoculation (described in the part entitled "Examples") of the aforementioned strain in the plant of interest.

Such an effect, which is surprisingly pronounced, is a unique characteristic of the strain described here.

The characteristics described above for the strain of the invention can also be present in derivatives of the strain.

Experiments performed by the inventor on the selected strain MUCL54911 have also demonstrated that this strain belongs to the vegetative compatibility group (VCG) IT6 or to the VCG that has the greatest diffusion of strains of *Aspergillus flavus* in Italian territory.

The strain MUCL54911 described here, and also the derivatives thereof, can also be formulated as a composition together with one or more diluents and/or carriers. Any diluent and/or carrier described in the prior art and considered suitable by a person skilled in the art for inclusion in the composition comprising the strain MUCL54911 is to be understood as included within the scope of the present invention. Merely by way of non-limiting example, the diluents and the carriers can be selected from the group comprising: water, media suitable for the cultivation of fungi, preservatives, additives, amino acids, salts, etc., materials of plant origin and/or products derived therefrom, any solid and/or liquid support capable of carrying the fungus. In particular, both diluents and carriers will be preferably sterile for the purpose of preventing the introduction of undesirable microorganisms into the composition.

Where it is indicated that the composition of the invention "may comprise" the strain MUCL54911, such a composition is understood also as "consisting of" this strain MUCL54911, and, optionally, a suitable carrier/diluent.

In one embodiment of the present composition, the strain is inoculated on a matrix or support that must be able to allow the strain to maintain all the characteristics associated therewith and described here. The matrix in which the strain MUCL54911 will be inoculated can be any matrix of natural, synthetic or also semi-natural/semi-synthetic origin considered to be suitable by a person skilled in the art in accordance with that indicated in the present description. The matrix/support is preferably sterile and derived from materials of plant origin, such as seeds or products obtained from the processing of the materials of plant origin, such as plant starches.

By way of example, the matrix can be a natural matrix selected primarily from plant materials obtained from plants such as sorghum, maize, soy and wheat.

In one embodiment, such plant material comprises seeds, such as wheat seeds and/or sorghum seeds.

As demonstrated in the section below entitled "Examples", the strain MUCL54911 described here has proven to be particularly effective if used as a biocontrol agent against the contamination by aflatoxins produced by toxigenic strains of *Aspergillus flavus and*/*or Aspergillus parasiticus.*

The use of the strain MUCL54911 as a biocontrol agent, for example in the field of agriculture, and specifically in the case of contamination by aflatoxins, which damage crops of maize, cotton and nuts, including pistachios, peanuts, walnuts, hazelnuts, almonds and chestnuts, is particularly advantageous. In fact, as noted above, the strain described here is able to reduce the contamination by aflatoxins produced by the toxigenic strains of *Aspergillus flavus* and/or *Aspergillus parasiticus* in a percentage between 75-100% in full field tests and/or is able to reduce the aflatoxin content in a percentage greater than 80% in crops contaminated by toxigenic strains of *Aspergillus flavus* and/or *Aspergillus parasiticus* with the method of pin bar inoculation.

The present invention also relates to a method for preventing and/or reducing and/or eliminating the contamination by aflatoxins produced by toxigenic strains of *Aspergillus flavus* and/or *Aspergillus parasiticus* in full field crops or in controlled environments, such as tunnels or greenhouses and soilless crops, comprising at least one step of distributing the strain MUCL54911 or of the composition as described above in the crop.

The method described here is preferably used to prevent and/or eliminate partially or wholly the contamination by aflatoxin B₁.

In particular, the step of distribution can be performed in accordance with any technique known to a person skilled in the art and suitable for allowing the diffusion of the biocontrol agent, or of the composition comprising same, in the field to be treated. By way of non-limiting example, the step of distribution can be performed using means commonly available for fertilisation of the crops, such as fertiliser spreaders. The distribution can also be performed by means of an application from the air and/or directly to the soil or in any case in accordance with techniques considered by a person skilled in the art as effective in guaranteeing the use of the strain MUCL54911 in accordance with that described here. The agricultural crops that can be subjected to the method described above are selected for example from the group comprising maize crops, cotton crops and nut crops, such as pistachios, peanuts, walnuts, hazelnuts, almonds and chestnuts.

In an embodiment of the present method, the strain MUCL54911 or spores thereof are inoculated prior to the step of distribution on a matrix, preferably a natural matrix, of the type described above.

The invention will be described hereinafter in some of its exemplary forms with the objective of illustrating that described in the present patent application. Such examples are in no way considered to limit the scope of protection conferred.

### EXAMPLES

### Example 1

### Efficacy of non-toxigenic strains in reducing the content of aflatoxin B₁ in maize grain.

Eighteen (18) Italian non-toxigenic strains of *Aspergillus flavus* were compared with the strain of *Aspergillus flavus* NRRL21882 in order to assess the ability of reducing the content of aflatoxin B₁ produced by the toxigenic strain of *Aspergillus flavus* AF13 in maize grain *in vitro.* The strain of *Aspergillus flavus* NRRL21882 is the active component of the commercial product AFLAGUARD® (U.S. patent 6306386 of 23 October 2001), whilst the strain AF13 is commonly used in laboratory tests and in field tests as a high producer of aflatoxins (Cotty, P. J. 1989. Virulence and cultural characteristics of two Aspergillus flavus strains pathogenic on cotton. Phytopathology 79, 808-814). The spores of each strain were collected using cotton buffers from crops 6 days old (31°C in the dark), suspended in sterile distilled water, and the concentration was determined as described by Probst et al. (Probst, C., Bandyopadhyay, R., Price, L. E., Cotty, P. J. 2011. Identification of atoxigenic Aspergillus flavus isolates to reduce aflatoxin contamination of maize in Kenya. Plant Dis. 95, 212-218). The concentration of the spores was adjusted to 10⁵ spores/ml. The intact maize grain was sterilised in hot water for 45s at 80°C (Mehl, H. L., Cotty, P. J. 2010. Variation in competitive ability among isolates of Aspergillus flavus from different vegetative compatibility groups during maize Infection. Phytopathology 100, 150-159) and distributed between sterile flasks (10 g of maize per 150 ml flask). The flasks were closed using gas-permeable stoppers (Bugstoppers; Whatman, Piscataway, NJ) to prevent the loss of moisture and so as to allow gaseous exchange. After sterilisation, the amount of moisture of the maize was quantified using an HB43 halogen moisture analyser (Mettler Toledo, Columbus, OH) and the volume of water in which the suspension of spores of the non-toxigenic and toxigenic strains were applied to the grain was adjusted to bring the content of water of the grain to 25%. The spores of the non-toxigenic and toxigenic strains were added to the flasks at the same time and stirred gently to allow the distribution over the grain. The same quantity of water was added to the controls, but containing just the spores of the toxigenic strains. The inoculated maize was incubated at 31°C for 7 days in the dark. The experimental design of the experiment was completely randomised with four replications. The experiment was repeated twice. At the end of the incubation period, the content of aflatoxin B₁ was quantified as described by Probst et al. (Probst, C., Bandyopadhyay, R., Price, L. E., Cotty, P. J. 2011. Identification of atoxigenic Aspergillus flavus isolates to reduce aflatoxin contamination of maize in Kenya. Plant Dis. 95, 212-218).

All the non-toxigenic strains were able to reduce the content of toxin in the grain compared with that produced by the toxigenic strain. In particular, the contamination by means of aflatoxin B₁ in the grain inoculated with just the toxigenic strain AF13 was 146 ppm, whilst in the grain co-inoculated with the strain AF13 and the strain MUCL54911 the content of aflatoxin was reduced by 83% (Table 1).

### Example 2

### Assessment of the efficacy of the non-toxigenic strain MUCL54911 in reducing the content of aflatoxin B₁ produced by 6 Italian toxigenic strains

The strain forming the basis of the present description and the non-toxigenic strain A2321 used as positive control were tested for the ability to reduce the content of aflatoxin B₁ produced by 6 different Italian toxigenic strains on living maize grain *in vitro.* The experiment was performed as described in Example 1 using 6 Italian toxigenic strains instead of the toxigenic strain AF13. The strain forming the basis of the present description and the strain A2321 were capable of reducing the contamination by aflatoxin B₁ in the living maize grain inoculated with 6 different toxigenic strains (Table 2). The contamination of the grain inoculated with just the toxigenic strains varied between 17 and 98 mg/kg. The extent to which the contamination in the content of aflatoxin B₁ was reduced by the strain MUCL54911 was between 60% and 78%, with similar reductions (from 60% to 68%) in the grain inoculated with 5 of the strains that produce aflatoxins and a significantly greater reduction (78%) in the grain inoculated with 1 of the strains. The ability of the strain MUCL54911 to reduce to the same extent the content of aflatoxin B₁ produced by various Italian toxigenic strains makes it surprisingly suitable for use as a bicontrol agent for reducing the contamination by aflatoxins in maize crops.

### Example 3

### Influence of the ratio between non-toxigenic and toxigenic strain in reducing the contamination by aflatoxin B₁.

The strain MUCL54911 was tested for the ability to reduce the content of aflatoxin B₁ produced by the toxigenic strain AF13 in two different non-toxigenic:toxigenic ratios on living maize grain *in vitro.* In addition, the strain NRRL21882 was used as positive control, this being an American strain known for its efficacy in reducing the contamination by aflatoxins. In this case too, the experiment was performed as described in Example 1, but using two different non-toxigenic:toxigenic ratios (1:1 and 1:4). In addition to the reduction in % of the content of aflatoxin B₁, the efficacy (E) of each strain was also calculated using the following formula: E = R/(A/A+T), where R is the reduction in % of aflatoxin B₁ and the denominator represents the percentage of the non-toxigenic strain in each treatment.

In particular, the strain MUCL54911 demonstrated a greater reduction (72%) (Table 3) in the content of aflatoxin B₁ in the maize grain inoculated with equal quantities of the non-toxigenic and toxigenic strains compared with the grain inoculated with a dose of the non-toxigenic strain equal to one quarter of the toxigenic strain. In addition, the strain MUCL54911 demonstrated a greater efficacy compared with the strain NRRL21882 when tested in the non-toxigenic:toxigenic ratio 1:4. This result surprisingly shows that the strain MUCL54911 increases its efficacy in reducing the content of aflatoxin B₁ as its concentration decreases compared with the toxigenic strain. Thus, even if it were found in lower concentrations than the toxigenic strains in the field, it would be able to produce an optimum result in terms of reduction of the concentration of aflatoxins.

### Example 4

### Production of cyclopiazonic acid

The non-toxigenic strain of *Aspergillus flavus* MUCL54911 forming the basis of the present description was tested on intact maize grain and on a synthetic Czapek Agar substrate (CZ) for the production of *cyclopiazonic* acid (CPA).

The CPA from the grain was extracted following the methodology described by Urano et al. (Urano, T., Trucksess, M. W., Beaver, R. W., Wilson, D. M., Dorner, J. W., Dowell, F. E. ,1992. Co-occurrence of cyclopiazonic acid and aflatoxins in corn and peanuts. J. AOAC Int. 75, 838-841) and was quantified as reported by Zamboni et al. (2001; Zambonin, C. G., Monaci, L., Aresta, A. 2001. Determination of cyclopiazonic acid in cheese samples using solid phase microextraction and high performance liquid chromatography. Food Chem. 75, 249-254). The methodology reported by Giorni e al. (2007; Giorni, P., Magan, N., Pietri, A., Bertuzzi, T., Battilani, P. 2007. Studies on Aspergillus section Flavi isolated from maize in northern Italy. Int. J. Food Microbiol. 113, 330-338) was instead followed for the extraction and the quantification of CPA from the culture medium CZ. The experiment was performed both cases with 4 replications.

In particular, the strain MUCL54911 did not produce remarkable quantities of *cyclopiazonic* acid (CPA) (Table 4) either on natural substrate (maize grain) or on synthetic substrate (CZ), by contrast with that observed for the strains used as positive control A2062, A2068 and A2092.

### Example 5

### Test on maize ears

The Italian non-toxigenic strain MUCL54911 forming the basis of the present description was tested on maize ears in full field for the ability to reduce the content of aflatoxin B₁ produced by a toxigenic strain using 2 inoculation methods (drop and pinhole).

The strain was assessed for the ability to reduce the content of aflatoxin B₁ produced by the toxigenic strain A2092 (VCG IT3) on maize ears. Two types of inoculation were performed on the maize ears. In the first (pinhole), the methodology described by Atehnkeng et al. (Atehnkeng, J., Ojiambo, P. S., Donner, M., Ikotun, T., Sikora, R. A., Cotty, P. J., Bandyopadhyay, R. 2008. Distribution and toxigenicity of Aspergillus species isolated from maize kernels from three agro-ecological zones in Nigeria. Int. J. Food Microbiol. 122, 74-84) was followed. After silking, a pinhole (3 mm in diameter) was made in each maize ear through the bracts up to a depth of approximately 5 mm before performing inoculation with 10 µl of a suspension of 10⁶ spores/ml of just one toxigenic strain (A2092) in the control or with the toxigenic and non-toxigenic strain, distributed at the same time, in the co-inoculation experiment. In the second type of inoculation (drop), the fungus strains were inoculated at the tip of the ears, at the point of exit of the silk, with 1 ml of a suspension of 10⁶ spores/ml of just one toxigenic strain (A2092) in the control or with the toxigenic and non-toxigenic strain at the same time in the co-inoculation experiment. The experiment, carried out in 2012 and 2013, was performed on 5 maize ears for each treatment. The aflatoxins were quantified following the methodology reported by Stroka et al. (2003; Stroka, J., von Holst, C., Anklam, E. 2003. Immunoaffinity column cleanup with liquid chromatography using post-column bromination for determination of aflatoxin B1 in cattle feed: collaborative study. J. AOAC Int. 86, 1179-1186). The isolates were recovered from the harvested grain by means of successive attributions to the VCGs.

The non-toxigenic strains MUCL54911 (IT6) and A2321 (IT19) were not able to reduce the content of aflatoxin B₁ produced by the toxigenic strain (A2092) when inoculated using the method in which a drop is deposited at the point of exit of the silk from the bracts. Therefore, this type of inoculation was not repeated in the second year (2013) of experimentation.

In 2012 the strains A2321 and MUCL54911, when co-inoculated with the toxigenic strain A2092 by means of the pinhole method, were able to reduce the content of aflatoxin B₁, respectively by 2.4% and by 93.2% (Table 5). In 2013 the strain A2321 was unable to reduce the content of aflatoxin B₁ produced by the toxigenic strain A2092, and in fact the amount of aflatoxin was considerably greater in the co-inoculation compared with the sole toxigenic strain (Table 5). By contrast, the strain MUCL54911 forming the basis of the present description reduced the content of aflatoxin B₁ by 98.3% when co-inoculated with the toxigenic strain A2092.

The experiment, performed for two years with similar results, demonstrates that the strain A2321, although able to reduce the content of aflatoxin more effectively in experiments performed in a laboratory, when tested in full field is unable to limit the production of aflatoxins from a toxigenic strain.

By contrast, the strain MUCL54911 forming the basis of the present description has proven to be able to significantly reduce the content of aflatoxin in both years considered.

### Example 6

### Tests in full field

The non-toxigenic strain MUCL54911 forming the basis of the present description and belonging to VCG IT6 and the strain A2321 belonging to VCG IT19 were tested in 8 fields of maize, measuring approximately 2 hectares each, to assess the efficacy in reducing the contamination by aflatoxin B₁ in natural inoculation conditions. The fields were located in the province of Verona (2 fields; VR1, VR2), Rovigo (3 fields; RO1, RO2, RO3), Mantova (2 fields, MN1, MN2) and Parma (PR). The experiment was organised with 3 replications of the treatment and of the control for each field. Upon harvesting, 10 ears of maize were sampled from each repetition and were shelled, and the grain was dried prior to being ground. The flour obtained was used to estimate the recovery of the 2 applied strains and to perform the analysis of aflatoxin B₁. In each repetition the aflatoxin was quantified by following the methodology reported by Stroka et al. (2011; Stroka, J., von Holst, C., Anklam, E., 2003. Immunoaffinity column cleanup with liquid chromatography using post-column bromination for determination of aflatoxin B1 in cattle feed: collaborative study. J. AOAC Int. 86, 1179-1186). The estimation of the recovery of the 2 applied strains was assessed on 30 isolates of *Aspergillus flavus* for each group (treated and control) by means of analysis of the vegetative compatibility that uses *nit* mutants. *Nit* mutants and the analysis of the vegetative compatibility were performed following the procedure described by Mauro et al. (2013, Mauro, A., Battilani, P., Callicott, K. A., Giorni, P., Pietri, A., Cotty, P. J. 2013. Structure of an Aspergillus flavus population from maize kernels in northern Italy. Int. J. Food Microbiol. 162, 1-7).

In particular, it was observed that in 4 of the analysed fields (MN1, MN2, RO1, RO2) the contamination by aflatoxin B₁ was less than the limit of detection of the analysis method used and also less than the limits enforced legally (Figure 1). In the remaining fields (RO3, PR, VR1 and VR2), where the contamination was between 9.97 µg/kg and 156.42 µg/kg, the reduction of the contamination by aflatoxin B₁ was generally between 83.3% and 99.6% (Figure 1).

The analysis of the vegetative compatibility for estimating the recovery of the 2 applied strains demonstrated on average that in the 8 fields analysed, the percentage of recovery of the strain MUCL54911 (IT6) in the treated group was 61.9% and that of strain A2321 was 30.0%. In the control group, where the 2 strains were not applied, the mean percentage of recovery in the 8 fields of the strain MUCL54911 (IT6) was 53.5% and that of the strain A2321 was 25.2%.

In particular in the 4 fields (RO3, PR, VR1 and VR2) where a reduction in the content of aflatoxin B₁ was obtained, the strain having the deposit number MUCL54911 was always recovered in a greater percentage compared with the strain A2321 (Figure 2).

Considering that the strain MUCL54911 was recovered on average in greater quantities than the strain A2321, both in the treated group and in the control group, we can conclude that the strain forming the basis of the present invention is able to adapt to the different agro-environmental conditions, and better compete and diffuse in the environment than the strain A2321 and is thus better suited for use in different environments and in different meteorological conditions

### Example 7

### Microsatellites

The genome of the strain MUCL54911 (IT6) forming the basis of the present patent and the strain A2321 (IT19) was amplified with 19 pairs of primers in order to identify the presence of microsatellites in accordance with the methodology described by Grubisha and Cotty (2009; Grubisha, L. C., Cotty, P. J. 2009. Twenty-four microsatellite markers for the aflatoxin-producing fungus Aspergillus flavus. Mol. Ecol. Resour. 9, 264-267).

The amplification of the genome of the strain MUCL54911 forming the basis of the present description demonstrated an allele profile characteristic as reported in Table 5. In particular, alleles were not identified for three loci (AF18, AF33, AF34), whereas the size in pairs of bases of each allele for the loci AF8, AF11, AF13, AF17, AF42, AF55 and AF66, which are polymorphs with the strain A2321, was, respectively, 141, 367, 150, 181 and 271.

### Example 8

### Genetic profile of the MAT genes

The genome of the strain MUCL54911 (IT6) forming the basis of the present patent was amplified by MAT genes as reported by Ramirez-Prado et al. (Ramirez-Prado, J. H., Moore, G. G., Horn, B. W., Carbone, I. 2008. Characterization and population analysis of the mating-type genes in Aspergillus flavus and Aspergillus parasiticus. Fungal Genet. Biol. 45, 1292-1299). In particular, the molecular analyses of the MAT genes showed that the strain MUCL54911 (IT6) forming the basis of the present patent has the allele MAT1-1.

## Claims

1. An atoxigenic *Aspergillus flavus* strain having deposit number MUCL54911.

2. A composition comprising the atoxigenic *Aspergillus flavus* strain according to claim 1 and one or more diluents and/or carriers.

3. The composition according to claim 3, wherein said strain is inoculated on a matrix.

4. The composition according to claim 3, wherein said matrix is a natural matrix and optionally, wherein said natural matrix comprises plant material obtained from plant selected form the group sorghum, maize, soy and wheat, said plant material optionally comprising wheat seeds and/or sorghum seeds.

5. Use of the atoxigenic *Aspergillus flavus* strain according to claim 1 or the composition according to any one of claims from 2 to 4 as biocontrol agents against the aflotoxin produced by toxigenic *Aspergillus flavus* strains and/or *Aspergillus parasiticus* strains contamination and wherein said aflatoxins optionally comprise B1, B2, G1, G2 aflatoxins or mixtures thereof.

6. Use according to claim 5, wherein said biocontrol is in agriculture.

7. Use according to claim 6, wherein said agriculture biocontrol comprises biocontrol in maize, peanut, pistachio, walnut, little nut, almond, chestnut, cotton crops.

8. A method for preventing and/or reducing and/or eliminating aflatoxins produced by toxigenic *Aspergillus flavus* strains and/or *Aspergillus parasiticus* strains in full field crops and/or in controlled environments and/or of in soilless crops comprising at least a step of distributing to the crop thereof the strain according to claim 1 or of the composition according to any one of claims from 2 to 4.

9. The method according to claim 8, wherein said aflatoxins comprise B1, B2, G1, G2 aflatoxins or mixtures thereof.

10. The method according to claim 8 or 9, wherein said crop is selected from the group comprising maize, peanut, pistachio, walnut, little nut, almond, chestnut, cotton crops.

11. A matrix inoculated with the atoxigenic *Aspergillus flavus* strain according to claim 1.

12. The matrix according to claim 11, wherein said matrix is a natural matrix.

13. The matrix according to claim 12, wherein said natural matrix comprises plant material obtained from plant selected form the group sorghum, maize, soy and wheat.

14. The matrix according to claim 13, wherein said plant material comprises wheat seeds and/or sorghum seeds.

## Patentansprüche

1. Nicht toxigener Stamm von *Aspergillus flavus* mit der Depotnummer MUCL54911.

2. Zusammensetzung mit dem nicht toxigenen Stamm von *Aspergillus flavus* gemäß Anspruch 1 und einem oder mehreren Verdünnern und/oder Trägern.

3. Zusammensetzung nach Anspruch 2, wobei der Stamm auf eine Matrix geimpft ist.

4. Zusammensetzung nach Anspruch 3, wobei die Matrix eine natürliche Matrix ist und wobei die natürliche Matrix optional Pflanzenmaterial umfasst, das von Pflanzen erhalten wird, die aus der Gruppe Hirse, Mais, Soja und Weizen ausgewählt sind, wobei das Pflanzenmaterial optional Weizensamen und/oder Hirsesamen umfasst.

5. Verwendung des nicht toxigenen Stammes von *Aspergillus flavus* nach Anspruch 1 oder der Zusammensetzung nach einem der Ansprüche 2 bis 4 als Mittel zum biologischen Schutz gegen das Aflatoxin, das durch Stämme von toxigenem *Aspergillus flavus* erzeugt wird und/oder einer Kontamination von Stämmen von *Aspergillus parasiticus,* wobei die Aflatoxine optional B1-, B2-, G1-, G2-Aflatoxine oder Mischungen davon umfassen.

6. Verwendung nach Anspruch 5, wobei die biologische Regelung in der Landwirtschaft stattfindet.

7. Verwendung nach Anspruch 6, wobei der landwirtschaftliche biologische Schutz einen biologischen Schutz von Mais, Erdnüssen, Pistazien, Walnüssen, kleinen Nüssen ("little nut"), Mandeln, Kastanien, Baumwollsaaten umfasst.

8. Verfahren zum Verhindern und/oder Vermindern und/oder Eliminieren von Aflatoxinen, die durch toxigene Stämme von *Aspergillus flavus* und/oder von Stämmen von *Aspergillus parasiticus* in vollen Feldsaaten und/oder in geregelten Umgebungen und/oder in erdelosen Saaten erzeugt werden, umfassend wenigstens einen Schritt zum Verteilen auf deren Saaten den Stamm nach Anspruch 1 oder die Zusammensetzung nach einem der Ansprüche 2 bis 4.

9. Verfahren nach Anspruch 8, wobei die Aflatoxine B1-, B2-, G1-, G2-Aflatoxine oder Mischungen davon umfassen.

10. Verfahren nach Anspruch 8 oder 9, wobei die Saat aus der Gruppe ausgewählt ist, die Mais, Erdnüsse, Pistazien, Walnüsse, kleine Nüsse ("little nut"), Mandeln, Kastanien, Baumwollsaaten umfasst.

11. Matrix, die mit dem nicht toxigenen Stamm von *Aspergillus flavus* nach Anspruch 1 geimpft ist.

12. Matrix nach Anspruch 11, wobei die Matrix eine natürliche Matrix ist.

13. Matrix nach Anspruch 12, wobei die natürliche Matrix Pflanzenmaterial umfasst, das von einer Pflanze erhalten wird, die aus der Gruppe Hirse, Mais, Soja und Weizen ausgewählt ist.

14. Matrix nach Anspruch 13, wobei das Pflanzenmaterial Weizensamen und/oder Hirsesamen umfasst.

## Revendications

1. Souche atoxigène d'*Aspergillus flavus* déposée sous le numéro MUCL54911.

2. Composition comprenant la souche atoxigène d'*Aspergillus flavus* selon la revendication 1 et un ou plusieurs diluants et/ou véhicules.

3. Composition selon la revendication 3, dans laquelle ladite souche est inoculée sur une matrice.

4. Composition selon la revendication 3, dans laquelle ladite matrice est une matrice naturelle et éventuellement, dans laquelle ladite matrice naturelle comprend du matériel végétal provenant d'une plante choisie dans le groupe du sorgho, du maïs, du soja et du blé, ledit matériel végétal comprenant éventuellement des semences de blé et/ou des semences de sorgho.

5. Utilisation de la souche atoxigène d'*Aspergillus flavus* selon la revendication 1 ou de la composition selon l'une quelconque des revendications 2 à 4 à titre d'agents de biocontrôle contre l'aflatoxine produite lors d'une contamination par des souches toxigènes d'*Aspergillus flavus* et/ou des souches d'*Aspergillus parasiticus* et dans laquelle lesdites aflatoxines comprennent éventuellement les aflatoxines B1, B2, G1, G2 ou leurs mélanges.

6. Utilisation selon la revendication 5, dans laquelle ledit biocontrôle s'exerce dans l'agriculture.

7. Utilisation selon la revendication 6, dans laquelle ledit biocontrôle agricole comprend le biocontrôle des cultures de maïs, arachides, pistaches, noix, petites noix, amandes, châtaignes, coton.

8. Méthode destinée à prévenir et/ou à réduire et/ou à éliminer les aflatoxines produites par des souches toxigènes d'*Aspergillus flavus* et/ou des souches d'*Aspergillus parasiticus* dans les cultures en plein champ et/ou dans des environnements contrôlés et/ou dans des cultures hors sol comprenant au moins une étape d'application à la culture de la souche selon la revendication 1 ou de la composition selon l'une quelconque des revendications 2 à 4.

9. Méthode selon la revendication 8, dans laquelle lesdites aflatoxines comprennent les aflatoxines B1, B2, G1, G2 ou leurs mélanges.

10. Méthode selon la revendication 8 ou 9, dans laquelle ladite culture est choisie dans le groupe comprenant les cultures de maïs, arachides, pistaches, noix, petites noix, amandes, châtaignes, coton.

11. Matrice inoculée avec la souche atoxigène d'*Aspergillus flavus* selon la revendication 1.

12. Matrice selon la revendication 11, dans laquelle ladite matrice est une matrice naturelle.

13. Matrice selon la revendication 12, dans laquelle ladite matrice naturelle comprend un matériel végétal obtenu à partir d'une plante choisie dans le groupe du sorgho, du maïs, du soja et du blé.

14. Matrice selon la revendication 13, dans laquelle ledit matériel végétal comprend des semences de blé et/ou des semences de sorgho.
